# EUROPEAN PATENT APPLICATION

(11) **EP 2 556 792 A1**
(43) Date of publication of application: **13.02.2013**
(21) Application number: 12005689.0
(22) Date of filing: 02.08.2012
(51) Int. Cl.: A61B 3/13, A61B 3/14, A61B 3/00, A61B 3/036, A61B 3/103, A61F 9/007

(54) **A microscope for ophtalmologic surgery**

(30) Priority: 11.08.2011 JP 2011175977
(71) Applicant: Kabushiki Kaisha Topcon, Tokyo 174-8580 (JP)
(72) Inventor: Kitajima,Nobuaki, Saitama 349-0212 (JP); Sato, Toshiaki, Tokyo (JP)
(74) Representative: Gagel, Roland

(57) **Abstract**

The microscope for ophthalmologic surgery 1 can perform astigmatism measurement for a patient's eye E by using a projection-image forming part 13 attached to a microscope 6. The projection-image forming part 13 is provided with multiple bright points (LEDs 131-i) for astigmatism measurement that are arranged in a ring, and a fixation target 131c arranged inside the ring-shaped arrangement. Based on a photographed image of the patient's eye E in a state in which the LEDs 131-i and the fixation target 131c are projected thereto, a displacement calculating part 92 detects the fixation state of the patient's eye E relative to the fixation target 131c. A controller 60 controls the LEDs 131-i and the fixation target 131c based on the detection result of the fixation state.

## Description

### [Field of the Invention]

The present invention relates to a microscope for ophthalmologic surgery.

### [Background of the Invention]

A microscope for ophthalmologic surgery, which can measure the astigmatic axis angles of a patient's eye and project the measurement results onto the patient's eye, is known (Refer to Japanese published unexamined application 2011-110172). The microscope for ophthalmologic surgery projects multiple bright points arranged in a circle onto the patient's eye, calculates the astigmatic axis angle based on the arrangement of these projection images, and is used for transplant surgeries of toric IOL (Intraocular Lens) for correcting astigmatism.

To effectively correct astigmatism, not only the astigmatic degree but also the astigmatic axis angle is important. Consequently, it is necessary to adjust the orientation of the lens with high accuracy when transplanting the toric IOL. The microscope for ophthalmologic surgery in Japanese published unexamined application 2011-110172 satisfies such needs. Furthermore, it is also advantageous in that the traditional method that directly marks the astigmatic axis angle on the patient's eye does not have to be followed.

In astigmatism measurement performed by this microscope for ophthalmologic surgery, the positional relationship between the patient's eye and the optical system is very important. For example, eye refraction measurement is preferably performed in the center of the cornea, but if the positions of the patient's eye and the optical system are misaligned, the measurement is performed at a positioned outside the center of the cornea and the accuracy of the measurement deteriorates. With conventional microscopes for ophthalmologic surgery, it has been difficult to effectively prevent such situations.

Furthermore, in other types of ophthalmologic devices, this problem is dealt with by performing alignment and eye fixation. For example, Japanese published unexamined application Hei 10-14878 describes technology that determines the suitability of the fixation state of a subject's eye based on the detection result of the pupil boundary of the subject's eye and on the detection result of an alignment target. Moreover, Japanese published unexamined application Hei 1-242029 describes technology that includes a fixation means having multiple elements and that selectively operates the elements so that the corneal reflection of visual targets and the subject's pupil become a prescribed positional relationship.

### [Prior Art Documents]

### [Patent Documents]

[Patent Document 1] Japanese published unexamined application 2011-110172
[Patent Document 2] Japanese published unexamined application Hei 10-14878
[Patent Document 3] Japanese published unexamined application Hei 1-242029

### [Summary of the Invention]

### [Problem to be Solved by the Invention]

However, due to the following reasons, it is not possible to apply the configurations of such other types of ophthalmologic devices to the above microscope for ophthalmologic surgery. Firstly, a conventional microscope for ophthalmologic surgery does not have an alignment optical system, and it may not be particularly necessary to have such a system. This is because the operator arranges the microscope at a desired position and performs surgery while changing the position as necessary. Consequently, it is not possible to apply the technology described in Japanese published unexamined application Hei 10-14878 to a microscope for ophthalmologic surgery.

Secondly, it is difficult to install the fixation means including multiple elements described in Japanese published unexamined application Hei 1-242029 onto a microscope for ophthalmologic surgery. That is, if one tries to install this fixation means on a microscope for ophthalmologic surgery, the position at which it is installed ends up being between the objective lens and the patient's eye, but if the fixation means is installed at this position, this blocks off the field of observation of the microscope. Consequently, with a microscope for ophthalmologic surgery, it is not possible to guide the line of sight of a patient's eye to a suitable direction for fixation using the technology described in Japanese published unexamined application Hei 1-242029.

In this way, because it is not possible to apply the technologies related to other types of ophthalmologic devices, with conventional microscopes for ophthalmologic surgery, it has not been possible to suitably adjust the positional relationship between the patient's eye and the optical system for astigmatism measurement.

Therefore, the objective of this invention is to provide a microscope for ophthalmologic surgery that allows the positional relationship between the patient's eye and the optical system for astigmatism measurement to be adjusted in a suitable manner.

### [Means for Solving the Problem]

A microscope for ophthalmologic surgery according to claim 1 comprises: an optical system comprising an imaging optical system that photographs a patient's eye; a main body part that stores at least part of the optical system; an attachment that is attached to the main body part and has both multiple bright points for astigmatism measurement arranged in a ring and a fixation target arranged inside the ring-shaped arrangement; a calculation part that calculates astigmatism information based on a first image obtained by using the imaging optical system to photograph the patient's eye while the multiple bright points are projected thereto; a detection part that detects the fixation state of the patient's eye relative to the fixation target based on a second image obtained by using the imaging optical system to photograph the patient's eye while the multiple bright points and the fixation target are projected thereto; and a controller that controls the multiple bright points and the fixation target based on the detection result of the fixation state.
An invention according to claim 2 is the microscope for ophthalmologic surgery according to Claim 1, wherein the controller includes a determination part that determines the quality of the fixation state based on the detection result of the fixation state, and if the fixation state is determined to be poor, the controller controls the multiple bright points and the fixation target to orient the line of sight of the patient's eye to the fixation target.
An invention according to claim 3 is the microscope for ophthalmologic surgery according to Claim 2, wherein if the determination part determines that the fixation state is good, the controller causes the calculation part to execute the calculation of the astigmatism information.
An invention according to claim 4 is the microscope for ophthalmologic surgery according to any of Claims 1 through 3, wherein the detection part detects the amount of displacement of the line of sight of the patient's eye relative to the fixation target as the fixation state, and the controller changes the details of the control of the multiple bright points and the fixation target in accordance with the amount of displacement.
An invention according to claim 5 is the microscope for ophthalmologic surgery according to any of Claims 1 through 4, wherein the detection part detects the direction of displacement of the line of the of the patient's eye relative to the fixation target as the fixation state, and the controller changes the details of the control of the multiple bright points and the fixation target in accordance with the direction of displacement.

### [Effect of the Invention]

According to such a microscope for ophthalmologic surgery, because it is possible to control the multiple bright points and the fixation target in accordance with the fixation state of the patient's eye, it is possible to adjust the positional relationship between the patient's eye and the optical system for astigmatism measurement in a suitable manner.

### [Brief Description of the Drawings]

Fig. 1 is a schematic drawing showing an example of the exterior configuration of the microscope for ophthalmologic surgery pertaining to the embodiment.
Fig. 2 is a schematic drawing showing an example of the configuration of a projection image forming part in the microscope for ophthalmologic surgery pertaining to the embodiment.
Fig. 3 is a schematic drawing showing an example of the configuration of a projection image forming part in the microscope for ophthalmologic surgery pertaining to the embodiment.
Fig. 4 is a schematic drawing showing an example of the configuration of an optical system in the microscope for ophthalmologic surgery pertaining to the embodiment.
Fig. 5 is a schematic drawing showing an example of the configuration of an optical system in the microscope for ophthalmologic surgery pertaining to the embodiment.
Fig. 6 is a schematic block diagram showing an example of the configuration of a control system in the microscope for ophthalmologic surgery pertaining to the embodiment.
Fig. 7A is a schematic explanatory drawing showing an action of the microscope for ophthalmologic surgery pertaining to the embodiment.
Fig. 7B is a schematic explanatory drawing showing an action of the microscope for ophthalmologic surgery pertaining to the embodiment.
Fig. 7C is a schematic explanatory drawing showing an action of the microscope for ophthalmologic surgery pertaining to the embodiment.
Fig. 7D is a schematic explanatory drawing showing an action of the microscope for ophthalmologic surgery pertaining to the embodiment.
Fig. 8A is a schematic explanatory drawing showing an action of the microscope for ophthalmologic surgery pertaining to the embodiment.
Fig. 8B is a schematic explanatory drawing showing an action of the microscope for ophthalmologic surgery pertaining to the embodiment.
Fig. 9A is a schematic explanatory drawing showing an action of the microscope for ophthalmologic surgery pertaining to the embodiment.
Fig. 9B is a schematic explanatory drawing showing an action of the microscope for ophthalmologic surgery pertaining to the embodiment.
Fig. 9C is a schematic explanatory drawing showing an action of the microscope for ophthalmologic surgery pertaining to the embodiment.
Fig. 10 is a flowchart showing an example of an operation of the microscope for ophthalmologic surgery pertaining to the embodiment.

### [Mode for Carrying Out the Invention]

An example of an embodiment of the microscope for ophthalmologic surgery, as related to the present invention, will be explained in detail with reference to the diagrams.

### [Configuration]

### [Exterior configuration]

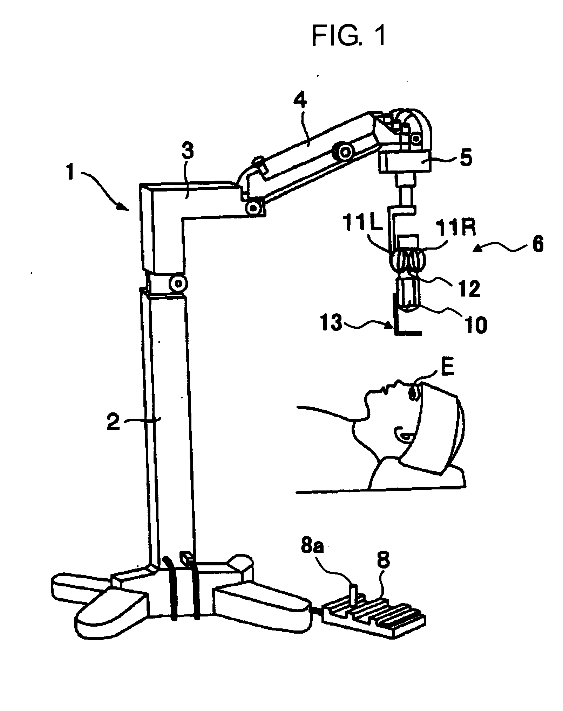

The exterior configuration of a microscope for ophthalmologic surgery 1, as related to the embodiment, will be explained with reference to Fig. 1. The microscope for ophthalmologic surgery 1 is composed of a pole 2, a first arm 3, a second arm 4, a driving device 5, a microscope 6, and a foot switch 8.

The driving device 5 is composed of an actuator such as a motor. The driving device 5 moves the microscope 6 up and down as well as horizontally, depending on the operation using an operating lever 8a of the foot switch 8. This makes it possible to move the microscope 6 3-dimensionally.

Various optical systems, drive systems, etc. are stored in a lens tube part 10 of the microscope 6. An inverter part 12 is provided in the top part of the lens tube part 10. When an observation image of a patient's eye is obtained as a reversed image, the inverter part 12 converts the observation image to an erect image. A left-and-right pair of eyepiece parts 11L and 11R is provided at the top part of the inverter part 12. An observer (such as an operator) can view the patient's eye with the both eyes by looking into the eyepiece parts 11L and 11R. The microscope 6 is an example of the "main body."

The microscope for ophthalmologic surgery 1 has a projection image forming part 13. The projection image forming part 13 is removable from the microscope 6 and forms a specified projection image on the patient's eye E by projecting luminous flux onto the patient's eye E. The projection image forming part 13 is an example of an "attachment."

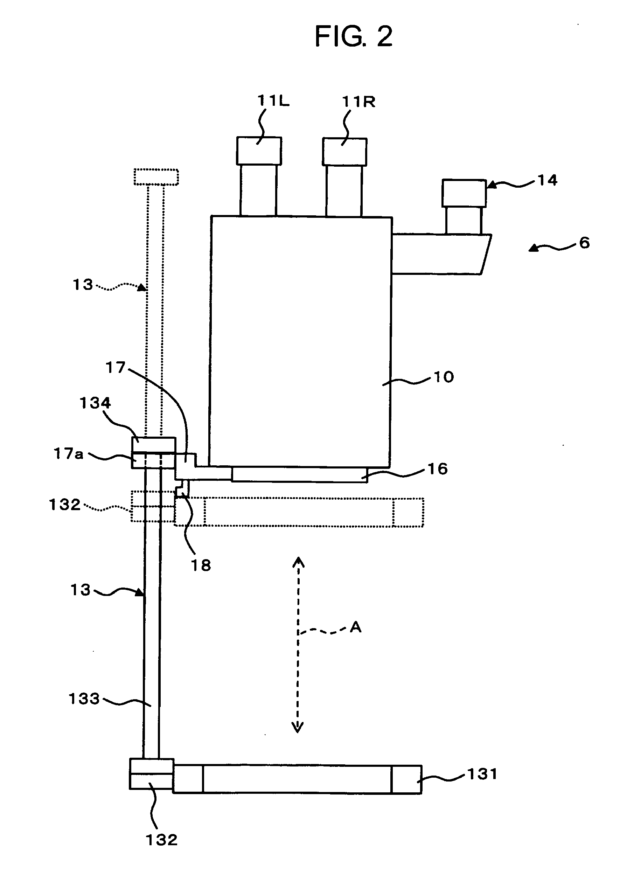

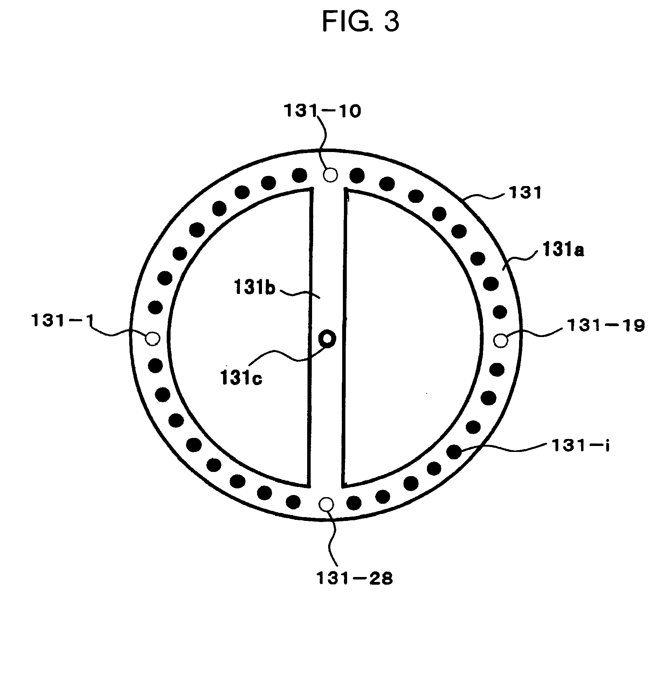

A configuration example of the projection image forming part 13 is shown in Fig. 2 and Fig. 3. The symbol 14 in Fig. 2 represents a photographing part. A TV camera 56, etc. that is discussed later is stored in the photographing part 14. Furthermore, Fig. 3 represents the configuration when a head part 131 of the projection image forming part 13 is viewed from the bottom (that is, from the side of the patient's eye E, in other words, from the side opposite to the lens tube part 10).

As indicated in Fig. 2 and Fig. 3, the head part 131 is a plate-like member. As indicated in Fig. 3, a toric periphery 131a and a fixation-light-source holder 131b, which is bridged in the direction of the diameter of the periphery 131a, are provided in the head part 131.

Multiple LEDs 131-i (i=1 to N) for astigmatism measurement are provided on the bottom surface of the periphery 131 a. A group of LED 131-i are placed in a ring. In this embodiment, 36 LEDs 131-i are provided at even intervals (N=36). That is, the group of LEDs 131-i are placed at intervals of 10 degrees with regard to the center position of the group of LEDs 131-i. In other words, when considering line segments that connect each LED 131-i and the center position, two line segments that relate to LED 131-i and the adjacent 131-(i+1) are crossed at the center position at an angle of 10°.

Each LED 131-i emits visible light. A group of LEDs 131-i can be composed such that all LEDs emit the same color and can also be composed to emit different colors. The latter case can be composed such that LEDs in the horizontal direction and the vertical direction in the group of LEDs 131-i output different colors from the LEDs in other directions. That is, it can be composed such that LEDs placed at astigmatic axis angles (astigmatic axis directions) 0 degrees, 90 degrees, 180 degrees, and 270 degrees (LED 131-1, 131-10, 131-19, 131-28 accordingly) output luminous flux of different colors from other LEDs 131-i (i≠1, 10, 19, 28). For example, red LEDs can be used for LEDs 131-1, 131-10, 131-19, 131-28 along with green LEDs for other LEDs 131-i (i≠1, 10, 19, 28). As a result, the horizontal direction and the vertical direction of the astigmatic axes can be easily identified.

Furthermore, only a part of LED 131-1, 131-10, 131-19, and 131-28 can be set up to output luminous flux of different colors from other LEDs. For example, only LED 131i corresponding to a 0 degree angle can be composed to output different colors than other LEDs (i≠1).

In addition, it is not necessary that all of the LEDs 131-1, 131-10, 131-19, and 131-28 are composed to output luminous flux of the same color (red in the example above). For example, red LEDs can be used for LEDs 131-1 and 131-19 while white LEDs can be used for LEDs 131-10 and 131-28 and green LEDs can be used for other LEDs 131-i (i≠1, 10, 19, 28). As a result, the horizontal direction and the vertical direction of the astigmatic axes can be easily identified.

Moreover, instead of making the horizontal direction and vertical direction identifiable by an output color of a light source such as above, other configurations can have a similar effect. For example, changing the brightness of the output light can make the directions identifiable.

On the bottom surface of the fixation-light-source holder 131b, a fixation target 131c for fixing the patient's eye E is provided. The fixation target 131c emits visible light. The fixation target 131c outputs light of a different color from the LEDs 131-i. From the perspective of the patient's eye E, the fixation target 131c is arranged inside the ring-shaped arrangement of the group of LEDs 131-i. In this embodiment, the fixation target 131 c is arranged in the central position of the ring-shaped arrangement.

The number of the fixation target 131c is not limited to 1 but any number can be provided. Furthermore, the fixation target 131c can be composed so as to be movable.

Moreover, the light source used for the head part does not have to be an LED and any device that can output light may be used. Furthermore, the multiple light sources placed in a ring do not have to be placed at even intervals. Further, because Fig. 3 is a diagram of the bottom surface, the placement order of the group of LEDs 131-i is set up in the opposite direction (reverse direction) from the direction set up for a general astigmatic axis. As a result, corneal reflection lights of luminous fluxes that re output from the group of LEDs 131-i (Purkinje images) are observed or photographed as the direction set up for a general astigmatic axis.

In addition, though 36 light sources are placed in a ring in this embodiment, the quantity is also arbitrary. It should be noted that when measuring the astigmatic axis angle of the patient's eye E based on the Purkinje image of luminous fluxes that are output by the group of LEDs 131-i, it is desirable that the quantity of light sources, which can ensure precision and accuracy of the measurements, are provided. When adopting a configuration that does not measure the stigmatic axis angles, there is no limit to the quantity of light sources in this sense.

Furthermore, depending on the precision required when the operator visually recognizes the orientation of the astigmatic axis angle and the principal meridian of toric IOL, the quantity of light sources can be properly set. For example, because light sources are placed at 10 degree intervals in this embodiment, the astigmatic axis angles can be presented in units of at least 10 degrees. In order to present the astigmatic axis angles, etc. with higher precision, the quantity of light sources, which corresponds to the precision (for example in units of 5 degrees, then 72 light sources), should be provided. The same can be said for lower precision cases.

Furthermore, though multiple light sources (the group of LEDs 131-i) are composed individually in this embodiment, this is not a restriction. For example, a display device (such as LCD (liquid-crystal display)) is provided on the bottom surface of the head part 131 and similar functionality can be obtained by displaying multiple bright points with this display device. In this case, each bright point corresponds to a light source.

An objective lens part 16 is provided at the bottom end of the lens tube part 10. An objective lens 15 (as stated below) is stored in the objective lens part 16. The supporting member 17 is provided in the vicinity of the objective lens part 16. The supporting member 17 is formed laterally from the objective lens part 16.

At the tip 17a of the supporting member 17, a vertically extending open hole is formed. An arm 133 is inserted into this open hole. The arm 133 is slidable inside the open hole. As a result, the arm 133 can be moved vertically (direction shown by the arrow A pointing at both sides in Fig. 2) with regard to the tip 17a. Here, the microscope 6 side is the top and the patient's eye E side is the bottom.

An anti-drop part 134 is provided at the top of the arm 133. The anti-drop part 134 is a plate-like member with a diameter bigger than the caliber of the open hole. As a result, the anti-drop part 134 prevents the arm 133 from falling from the tip 17a.

A head connecting part 132 is provided at the bottom end of the arm 133. The head connecting part 132 connects the head part 131 to the arm 133 such that the surface with LEDs 131-i face the bottom.

With such a configuration, the head part 131, the head connecting part 132, the arm 133, and the anti-drop part 134 (that is, the projection image forming part 13) can move freely and vertically with regard to the tip 17a. The projection image forming part 13 is moved by, for example, a user holding the arm 133, etc. Furthermore, by using a driving means such as a motor, the projection image forming part 13 can be composed such that it is moved electrically.

A connection hook 18 is provided on the bottom surface of the supporting member 17. The connection hook 18 is composed such that it can be engaged to an engaging part (not shown) of the projection image forming part 13. This engaging part is, for example, provided in the head connecting part 132. When the projection image forming part 13 is moved to the top, the engaging part engages the connection hook 18 to stop the vertical movement of the projection image forming part 13. This engagement relationship can be released by a specified operation (for example by pressing a specified button). The configurations of the connection hook 18 and the engaging part are optional.

With the above configuration, the group of LEDs 131-i is maintained such that they can move in the direction of the optical axis of the objective lens 15. Furthermore, the fixation target 131c is placed in the optical axis of the objective lens 15.

### [Configuration of the optical system]

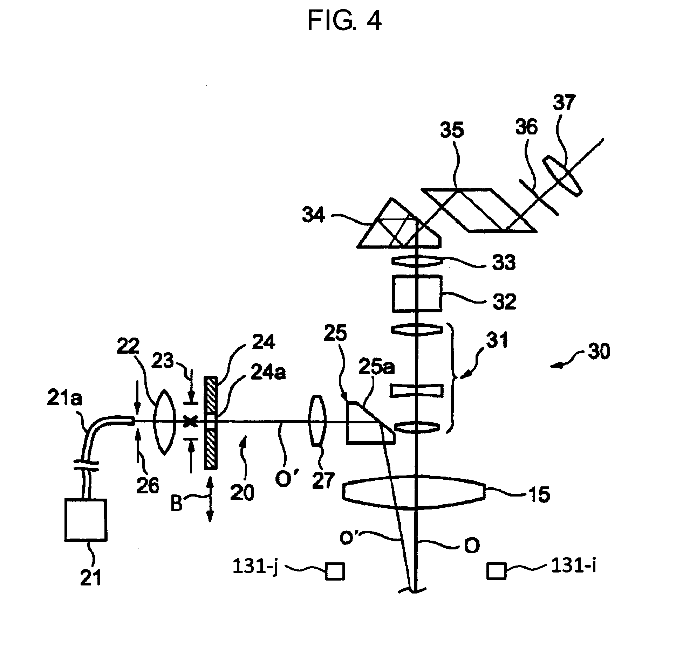

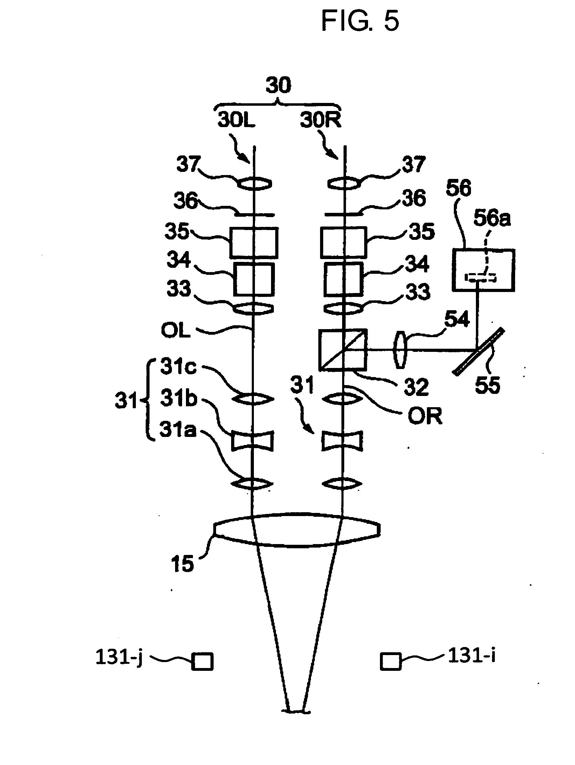

The optical system of the microscope for ophthalmologic surgery 1 will be explained next, with reference to Fig. 4 and Fig. 5. Here, Fig. 4 is a diagram, in which the optical system is viewed from the operator's left side. Moreover, Fig. 5 is a diagram, in which the optical system is viewed from the operator side. It should be noted that in addition to the configurations indicated in Fig. 4 and Fig. 5, an optical system (microscope for an assistant) for an assistant of the operator to observe the patient's eye E can be provided.

In this embodiment, directions such as top-and-bottom, left-and right, and front-and-back are directions viewed from the operator side unless otherwise specified. Moreover, with regards to the vertical direction, the direction from the objective lens 15 to the observation object (patient's eye E) is considered the lower direction while the opposite direction is considered the upper direction. Because patients generally go through an operation facing up, the top-and-bottom direction and the vertical direction become the same.

The group of LEDs 131-i is provided at the lower position of the objective lens 15 (that is, at the position between the objective lens 15 and the patient's eye E). Only LED 131-i and 131-j (i, j=1 to N, i≠j), which are positioned at both ends when viewed from the direction of this view point, are indicated in Fig. 4 and Fig. 5.

Moreover, a gap between the objective lens 15 and the patient's eye E means a gap between the position (height) of the objective lens 15 and the position (height) of the patient's eye E vertically (thus positions in the horizontal and front-and-back directions are not considered). Though the group of LEDs 131-i is placed in a ring, the diameter of this ring can be set arbitrarily as long as the size of an image of the light (bright point image) from the entire group of LEDs 131-i is projectable to the cornea of the patient's eye E.

An observation optical system 30 will be explained. A symmetrical pair of observation optical systems 30 is provided as indicated in Fig. 5. The observation optical system 30L on the left will be referred to as a left observation optical system and the observation optical system 30R will be referred to as a right observation optical system. A symbol OL indicates an optical axis (observation optical axis) of the left observation optical system 30L and a symbol OR indicates an optical axis (observation optical axis) of the right observation optical system 30R. The observation optical systems 30L and 30R on the left and right are arranged such that an optical axis O of the objective lens 15 is located in the middle of the optical axes OL and OR.

As before, each of the observation optical systems 30L and 30R on the left and right has a zoom lens system 31, a beam splitter 32 (only the right observation optical system 30R), an imaging lens 33, an image-erecting prism 34, a pupil-distance-adjusting prism 35, a field diaphragm 36, and an eyepiece lens 37.

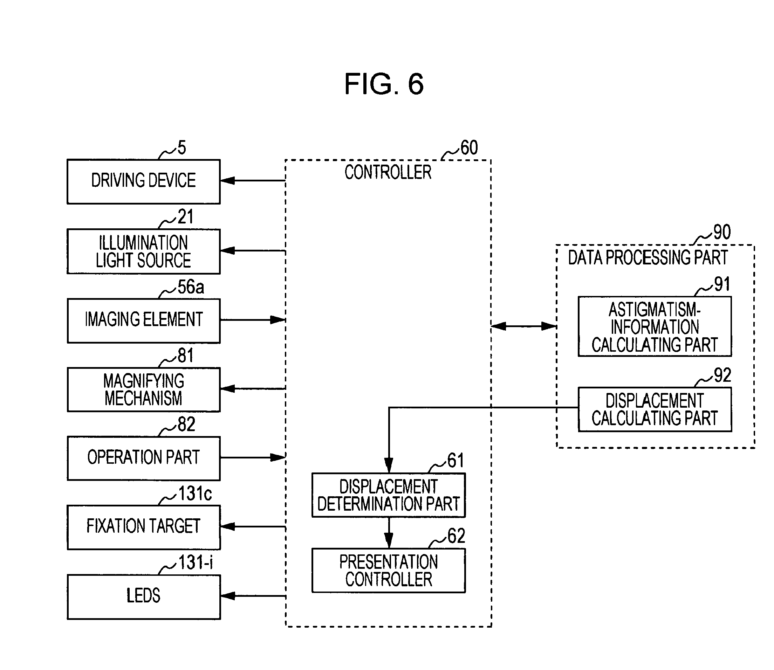

The zoom lens system 31 contains multiple zoom lenses 31a, 31b, and 31c. Each zoom lens 31 a to 31 c is movable in the direction of the observation optical axis OL (or the observation optical axis OR) by the magnifying mechanism 81 that will be mentioned later (refer to Fig. 6). As a result, the magnification is changed when observing or photographing the patient's eye E.

The beam splitter 32 of the right observation optical system 30R separates the part of the observation light that has been guided along the observation optical axis OR from the patient's eye E and leads to the TV camera imaging system. The TV camera imaging system comprises an imaging lens 54, a reflecting mirror 55, and a TV camera 56. The TV camera imaging system is stored in the photographing part 14. An optical element that is placed in the optical path from objective lens 15 to the TV camera 56 (imaging element 56a) is an example of an "imaging optical system."

The TV camera 56 is equipped with an imaging element 56a. The imaging element56a is, for example, composed of a CCD (Charge Coupled Devices) image sensor, CMOS (Complementary Metal Oxide Semiconductor) image sensor, etc. As the imaging element 56a, an element that has a two-dimensional light-receiving surface (that is, an area sensor) is used.

When using the microscope for ophthalmologic surgery 1, the light-receiving surface of the imaging element 56a is placed, for example, at an optically conjugated position with the corneal surface of the patient's eye E or at an optically conjugated position that is deep from the corneal apex by half of a corneal curvature radius.

The image-erecting prism 34 converts a reversed image to an erect image. The pupil-distance-adjusting prism 35 is an optical element for adjusting the distance between left and right observation lights in accordance with the pupil distance of the operator (the distance between left and right eyes). The field diaphragm 36 restricts the operator's field of vision by defilading the surrounding area in a cross-section of the observation light.

An illumination optical system 20 is explained next. As indicated in Fig. 4, the illumination optical system 20 comprises an illumination light source 21, an optical fiber 21a, an emission diaphragm 26, a condensing lens 22, an illumination field diaphragm 23, a slit plate 24, a collimator lens 27, and an illumination prism 25.

The illumination field diaphragm 23 is provided at a position that is optically conjugate to the front focal position of the objective lens 15. Furthermore, a slit hole 24a of the slit plate 24 is formed at the optically conjugate position against the front focal position.

The illumination light source 21 is provided outside the lens tube part 10 of the microscope 6. One end of the optical fiber 21a is connected to the illumination light source 21. The other end of the optical fiber 21a is placed at a position facing the condensing lens 22 inside the lens tube part 10. Illumination light that is output from the illumination light source 21 enters the condensing lens 22 after being guided by the optical fiber 21 a.

The emission diaphragm 26 is provided at a position facing the exit end (fiber edge on the condensing lens 22 side) of the optical fiber 21 a. The emission diaphragm 26 works such that some area of the exit end of the optical fiber 21a is covered. When the covered area is changed by the emission diaphragm 26, the exit area of the illumination light is changed. As a result, for example, the projection angle of the illumination light, that is the angle formed by the incident direction of the illumination light toward the patient's eye E and the optical axis O of the objective lens 15, can be changed.

The slit plate 24 is formed by a discoidal member with a light blocking effect. A transparent part that is composed of multiple slit holes 24a with shapes corresponding to the shape of the reflective surface 25a of the illumination prism 25 is provided on the slit plate 24. The slit plate 24 is moved in a direction orthogonal to an illumination optical axis O' (in the direction shown by the arrow B pointing at both sides in Fig. 4) by a drive mechanism (not drawn). As a result, the slit plate 24 is inserted into and removed from the illumination optical axis O'.

The collimator lens 27 turns the illumination light that has passed through the slit hole 24a into parallel luminous flux. The illumination light that has been turned into the parallel luminous flux is reflected by the reflective surface 25a of the illumination prism 25 and projected onto the patient's eye E via the objective lens 15. (Some of) the illumination light projected onto the patient's eye E is reflected by the cornea. The reflected light (sometimes referred to as observation light) of the illumination light by the patient's eye E enters the observation optical system 30 via the objective lens 15. Due to such a configuration, a magnified image of the patient's eye E becomes observable.

### [Configuration of the control system]

The control system of the microscope for ophthalmologic surgery 1 will be explained with reference to Fig. 6. Moreover, the control system is partially shown in Fig. 6. The drive mechanism for the slit plate 24, etc. is omitted.

### (Controller)

The control system of the microscope for ophthalmologic surgery 1 is composed around the controller 60. The controller 60 is provided at an arbitrary part of the microscope for ophthalmologic surgery 1. Furthermore, aside from the configuration indicated in Fig. 1, a computer and/or a circuit board can be provided and used as the controller 60. The controller 60 is composed of a microprocessor and a storage device similar to a normal computer.

The controller 60 controls each part of the microscope for ophthalmologic surgery 1. In particular, the controller 60 controls the driving device 5, the illumination light source 21, the magnifying mechanism 81, the group of LEDs 131-i, the fixation target 131c, etc. The driving device 5 moves the microscope 6 3-dimensionally after being controlled by the controller 60. The magnifying mechanism 81 moves each of the zoom lenses 31a, 31b, and 31c of the zoom lens system 31 after being controlled by the controller 60. For example, a pulse motor is provided in the driving device 5 and the magnifying mechanism 81. The controller 60 controls the motion by sending pulse signals to each pulse motor.

The controller 60 is provided with a displacement determination part 61 and a presentation controller 62. The displacement determination part 61 determines the fixation state of the patient's eye E obtained by the data processing part 90. The displacement determination part 61 is an example of the "determination part". The presentation controller 62 controls the LEDs 131-i and the fixation target 131c based on the determination result. Details of the operations of the displacement determination part 61 and the presentation controller 62 are described later.

### (Operation part)

The operation part 82 is used by an operator, etc. to operate the microscope for ophthalmologic surgery 1. The operation part 82 includes all types of hardware keys (buttons, switches, etc.) that are provided in a case, etc. of the microscope 6 as well as the foot switches 8. Furthermore, when a touch panel display and GUI are provided, various types of software keys displayed on these are included in the operation part 82.

### (Data processing part)

The data processing part 90 executes various types of data processing. The data processing part 90 is provided with an astigmatism-information calculating part 91 and a displacement calculating part 92.

### (Astigmatism-information calculating part)

Based on a photographed image (first image) of the patient's eye in a state in which the luminous fluxes from the LEDs 131-i are projected to the cornea, the astigmatism-information calculating part 91 calculates the astigmatic axis direction of the patient's eye E. Here, the astigmatic degree of the patient's eye E may also be calculated. This process includes a similar calculation process as that of a conventional keratometer, etc.

### (Displacement calculating part)

Based on a photographed image (second image) of the patient's eye E in a state in which the LEDs 131-i and the fixation target 131c are projected thereto, the displacement calculating part 92 detects the fixation state of the patient's eye E relative to the fixation target 131 c. The fixation state of the patient's eye E relative to the fixation target 131 c refers to the relationship between the direction of the line of sight of the patient's eye E and the position of the fixation target 131 c. The displacement calculating part 92 is an example of the "detection part".

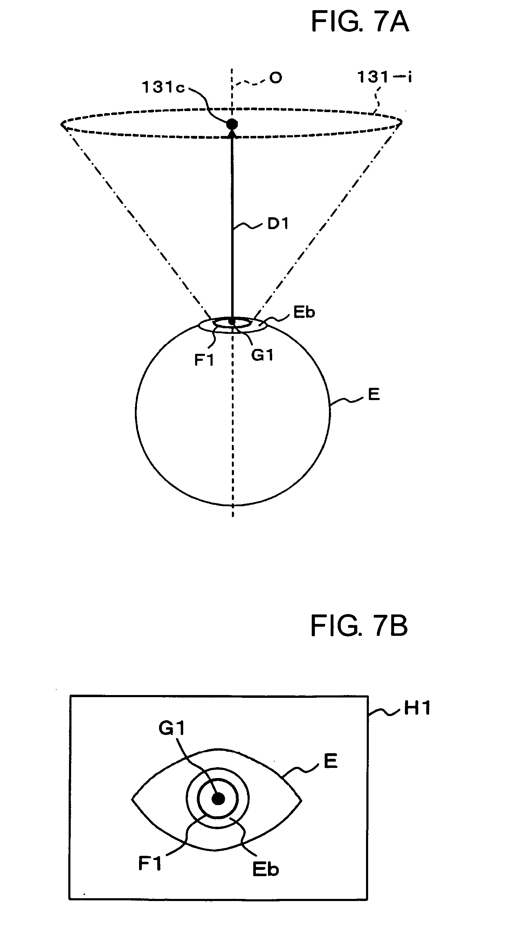

Examples of processes executed by the displacement calculating part 92 are described with reference to Fig. 7A through Fig. 9C. Fig. 7A shows a state in which the axis of the patient's eye E matches the optical axis O of the objective lens 15 and the line of sight D1 of the patient's eye E is oriented to the fixation target 131c. Here, the symbol Eb indicates the iris (dark part of an eye) (or pupil) of the patient's eye E, the symbol F1 indicates the projected image (ring-shaped image) of lights from the LEDs 131-i, and the symbol G1 indicates the projected image of light from the fixation target 131c.

The condition of "the axis of the patient's eye E matching the optical axis O of the objective lens 15" corresponds to the state of "matching alignment" in other types of ophthalmologic devices. As with the microscope for ophthalmologic surgery 1 of this embodiment, typical microscopes for ophthalmologic surgery are not provided with an alignment function. Consequently, this type of state is achieved by the operator carefully adjusting the position of the microscope 6 relative to the patient's eye E. On the other hand, the condition of "the line of sight D1 of the patient's eye E being oriented to the fixation target 131c" corresponds to the state of the "fixation correct" or the "fixation being achieved".

The state shown in Fig. 7A is an ideal state for astigmatism measurement, but because the careful adjustments described above are required, it is not very realistic. In astigmatism measurement using other types of ophthalmologic devices (autorefractometers or keratometers), measurement is performed in this state by performing alignment and eye fixation.

Fig. 7B shows a photographed image H1 of the patient's eye E in the state shown in Fig. 7A. In the following, the subject and the photographed image may be viewed as being the same. Because the axis of the patient's eye E and the optical axis O of the objective lens 15 match, the patient's eye E is depicted in the center of the frame of the photographed image H1. Moreover, because the eye fixation is correct, the projected image G1 of the fixation target 131c is depicted in the center of the frame (i.e., in the central position of the iris Eb). Based on the arrangement relationship between the LEDs 131-i and the fixation target 131c, the projected image G1 of the fixation target 131c is depicted in the center of the ring image F1.

The displacement calculating part 92 detects the outline of the iris EB depicted in the photographed image H1. This process includes, for example, edge detection based on pixel values of the photographed image H1. The iris (dark part of an eye) is composed of the pupil and the iris, and because the pupil is depicted more darkly (at a lower luminance) than the iris, the outline of the pupil can also be detected using a similar edge detection process. The edge detection process may include an edge-emphasizing process using a spatial filter or binarization.

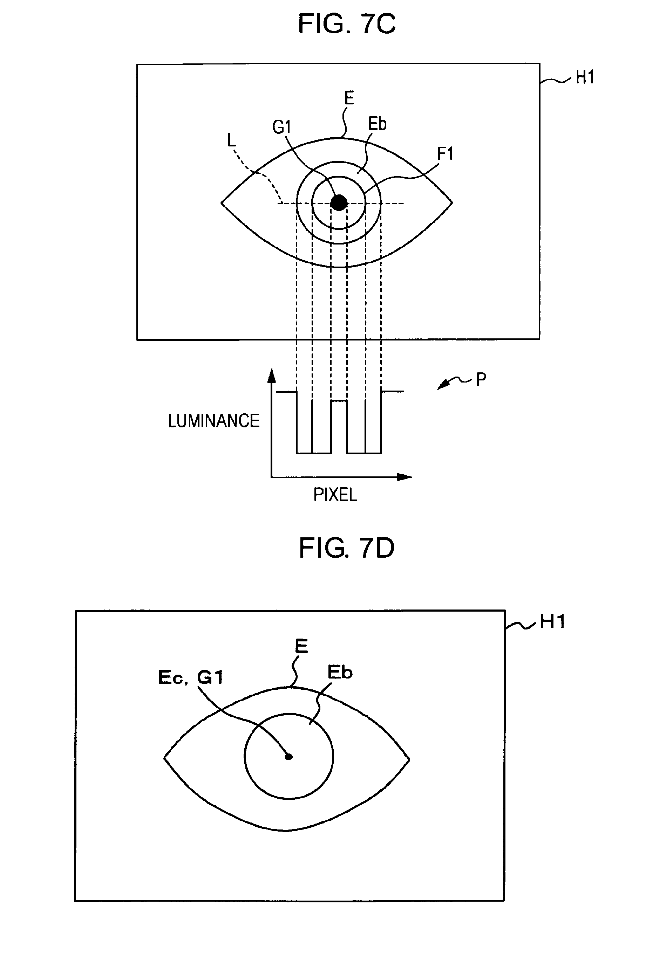

Fig. 7C shows a specific example of a process for detecting the outline of the iris Eb. In Fig. 7C, the image of the pupil and changes in luminance values related to the pupil are omitted. The displacement calculating part 92 creates a luminance profile P of pixels positioned on horizontal lines L in the photographed image H1 and performs a thresholding process of the luminance values to identify pixels corresponding to the outline of the iris Eb. By performing this process for each horizontal line, a two-dimensional distribution of the pixels corresponding to the outline of the iris Eb is obtained.

Furthermore, based on the detected outline of the iris Eb, the displacement calculating part 92 obtains the center of the iris Eb. This process is based on, for example, obtaining an approximate ellipsoid of the detected outline and obtaining the center of the approximate ellipsoid. Furthermore, the center of gravity, etc. may be obtained instead of the center. Moreover, if detecting the outline of the pupil, the center etc. of the pupil is obtained.

Moreover, the displacement calculating part 92 detects the projected image G1 of the fixation target 131 c depicted in the photographed image H1. Because the projected image G1 is an image obtained by receiving the reflected light of the fixation target 131 c, it is depicted with a high luminance as shown in Fig. 7C. Consequently, by identifying such high-luminance pixels using a thresholding process, etc., the projected image G1 is detected. The ring image F1 is also depicted with a high luminance, but it is possible to differentiate the projected image G1 and the ring image F1 by analyzing the arrangement (shape) of the image, etc. Moreover, this differentiation may be performed based on differences in the colors of the projected image G1 and the ring image F1. Moreover, it is also possible to perform this differentiation based on differences in luminance based on these differences in color.

Furthermore, the displacement calculating part 92 obtains the displacement between the center of the iris Eb and the projected image G1 of the fixation target 131c. As the position of the projected image G1, the center position or the position of the center of gravity of the projected image G1 is used. Fig. 7D shows the position relationship between the iris center and the projected image G1 in the case of Fig. 7A. In this case, the iris center Ec and the projected image G1 match. That is, the calculated displacement Δ is 0. It is rare for the displacement Δ to be 0 even if the fixation is correct, and there is a slight displacement.

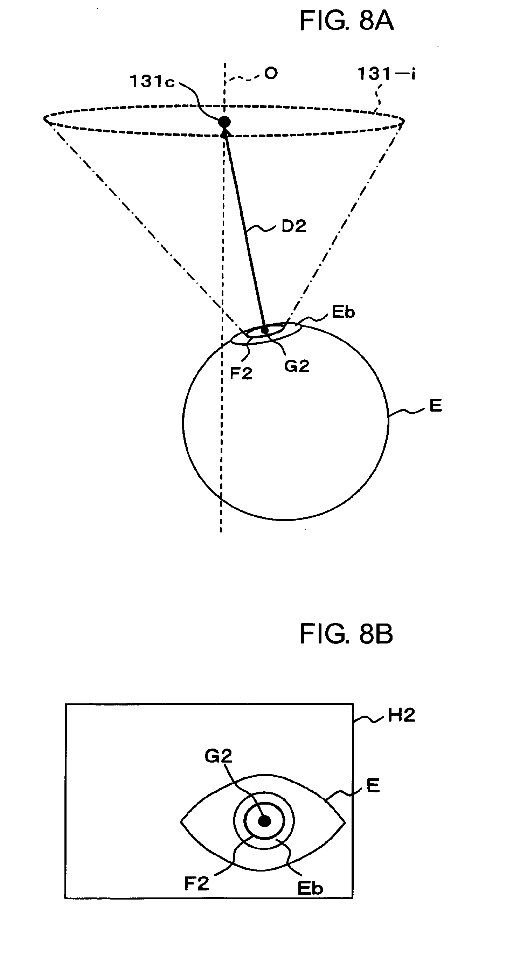

Fig. 8A shows a state in which the axis of the patient's eye E does not match the optical axis O of the objective lens 15 and the line of sight D2 of the patient's eye E is oriented toward the fixation target 131 c. This corresponds to a state in which "the alignment is not correct but eye fixation is achieved". With the microscope for ophthalmologic surgery 1, because an alignment function is not provided, it is realistic for astigmatism measurement to be performed in this state (details are described later).

Fig. 8B shows a photographed image H2 of the patient's eye E in the state shown in Fig. 8A. Because the axis of the patient's eye E does not match the optical axis O of the objective lens 15, the patient's eye E is depicted at a position outside the center of the frame of the photographed image H2. On the other hand, because the eye fixation is correct, the projected image G2 of the fixation target 131c is depicted in the central position of the iris Eb. The projected image G2 of the fixation target 131 c is depicted in the center of the ring image F2.

The displacement calculating part 92 analyzes the photographed image H2 and obtains the displacement between the center of the iris Eb and the projected image G2 of the fixation target 131 c. In the case shown in Fig. 8A, as with the case shown in Fig. 7A, because the iris center Ec matches the projected image G2, the displacement Δ is calculated to be 0.

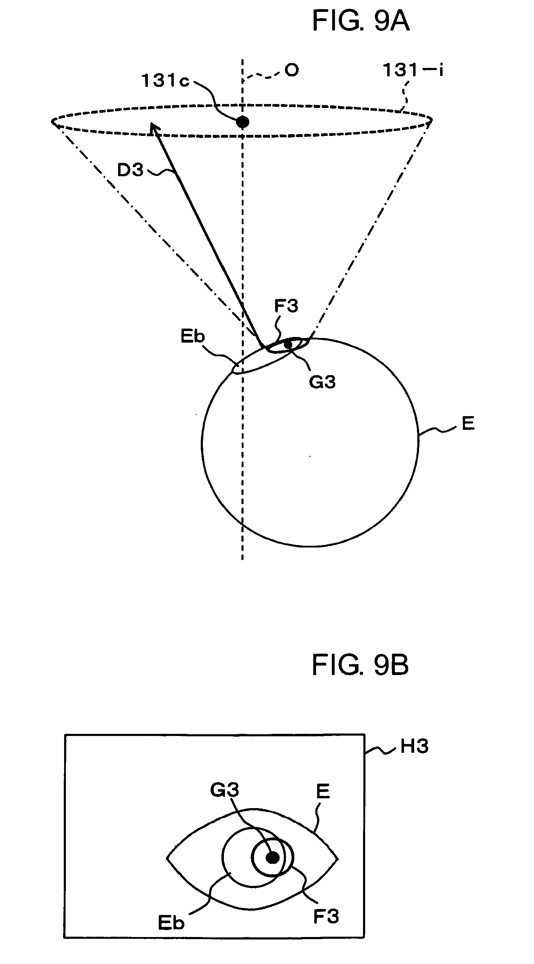

Fig. 9A shows a state in which the axis of the patient's eye E does not match the optical axis O of the objective lens 15 and the line of sight D3 of the patient's eye E is not orientated to the fixation target 131c. This corresponds to a state in which "neither the alignment nor the eye fixation are correct".

Fig. 9B shows a photographed image H3 of the patient's eye E in the state shown in Fig. 9A. Because the axis of the patient's eye E does not match the optical axis O of the objective lens 15, the patient's eye E is depicted at a position outside the center of the frame of the photographed image H3. Moreover, because the eye fixation is not correct, the projected image G3 of the fixation target 131c is depicted at a position away from the central position of the iris Eb. The projected image G3 of the fixation target 131c is depicted in the center of a ring image F3.

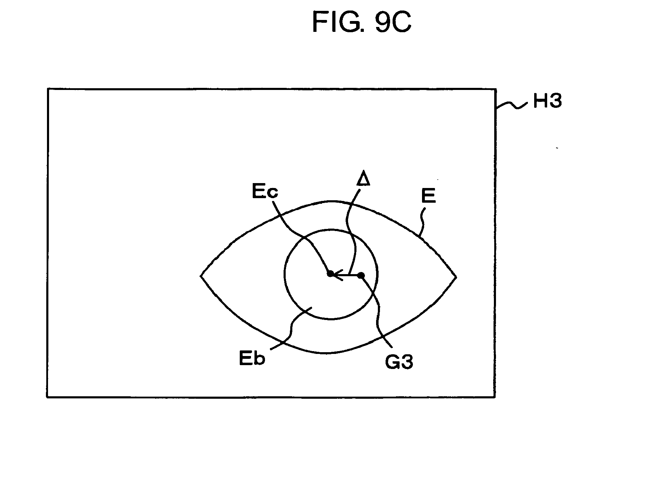

The displacement calculating part 92 analyzes the photographed image H3 and obtains the displacement between the center of the iris Eb and the projected image G3 of the fixation target 131c. In the case shown in Fig. 9A, the iris center Ec and the projected image G3 do not match, and a displacement Δ of "≠ 0" is calculated (refer to Fig. 9C). This may be the distance in actual space based on considerations of the imaging magnification, etc., or it may be a virtual distance based on pixel count, etc. Here, the displacement Δ of the iris center relative to the projected image of the fixation target 131c is used, but conversely, the displacement of the projected image relative to the iris center may be used. Moreover, the same applies for cases in which the pupil center is used instead of the iris center.

The displacement Δ calculated by the displacement calculating part 92 is sent to the displacement determination part 61. The displacement determination part 61 determines the quality of the fixation state based on the displacement Δ. This process is, for example, a thresholding process of the absolute value of the displacement Δ (i.e., the amount of displacement), or in other words, a comparison of the amount of displacement with a prescribed threshold value. If the amount of displacement is equal to or greater than the threshold value, the displacement determination part 61 determines that "the fixation state is poor", and if the amount of displacement is less than the threshold value, it determines that "the fixation state is good". The displacement determination part 61 sends this determination result (particularly determination results indicating that the fixation state is poor) to the presentation controller 62.

### (Presentation controller)

The presentation controller 62 controls the LEDs 131-i and the fixation target 131 c based on the results of the determination regarding the fixation state made by the displacement determination part 61. In particular, if the fixation state is determined to be poor, the presentation controller 62 controls the LEDs 131-i and the fixation target 131 c so that the line of sight of the patient's eye E is oriented to the fixation target 131c. This guides the line of sight of the patient's eye E to the fixation target 131c. A specific example of this process is described below.

As described previously, because the displacement calculating part 92 detects the fixation state based on a photographed image of the patient's eye E while the LEDs 131-i and the fixation target 131c are projected thereto, at this stage, each of the LEDs 131-i and the fixation target 131 c are on. First, the presentation controller 62 identifies the LED 131-j corresponding to the direction of the displacement obtained by the displacement calculating part 92. Next, the presentation controller 62 turns off the bright points other than the identified LED 131-j and the fixation target 131c (i.e., each of the LEDs 131-i, where i ≠ j). As a result, only the LED 131-j, which is in the direction of the line of sight of the patient's eye E, and the fixation target 131 c are on. As described previously, the LED 131-j and the fixation target 131c have different colors. In this state, the operator instructs the patient to look at the bright point of a prescribed color (i.e., the fixation target 131c). Because the bright point (LED 131-j) that is currently in the direction of the line of sight is not of this prescribed color, the patient moves their line of sight and seeks the bright point of the prescribed color. Because only the LED 131-j and the fixation target 131c are on, it is easy to find the fixation target 131c.

The method of guiding the line of sight is not limited to this. Generally, it is possible to guide the line of sight toward the fixation target 131 c by using various biological characteristics such as mental and psychological characteristics or visual characteristics. For example, it is possible to make the fixation target 131c blink or to increase the emission intensity. Moreover, it is possible to gradually guide the line of sight toward the fixation target 131 c by sequentially turning on and off multiple LEDs 131-i.

Moreover, the presentation controller 62 can change the details of the control of the LEDs 131-i and the fixation target 131c in accordance with the amount of displacement or the direction of displacement of the line of sight of the patient's eye E relative to the fixation target 131c. This process may be performed when the fixation state is determined to be good or when the fixation state is determined to be poor.

As an example of control performed when the fixation state is determined to be good, it is possible to notify the operator that the fixation state is good by temporarily changing the emission intensity of the LEDs 131-i. Moreover, if the fixation state has been determined to be good but the amount of displacement thereof is close to the threshold value, the lighting state of the LEDs 131-i and/or the fixation target 131c may be changed to provide notification of the risk that fixation misalignment may occur. Similarly, if the fixation state is determined to be good but the amount of displacement is close to the threshold value, the emission intensity of the LED(s) 131-i in the direction opposite from the direction of displacement and/or the fixation target 131 c may be increased to guide the line of sight toward the fixation target 131 c.

As an example of control performed when the fixation state is determined to be poor, it is possible to guide the line of sight to the fixation target 131c by, for example, changing the emission intensity of the fixation target 131c in accordance with the amount of displacement (e.g., by making the amount of displacement and the emission intensity proportionate to each other). Moreover, if the amount of displacement is very large (e.g., if the amount of displacement is equal to or greater than another threshold value greater than the above), because there is a possibility that the fixation target 131c is not in the field of vision at all, it is possible to try and make the line of sight return by turning on any of the LEDs 131-i, particularly the LED(s) 131-i corresponding to the direction of displacement, and then turning on the fixation target 131c to guide the line of sight to the fixation target 131 c.

Moreover, to notify the operator of the fixation state, it is possible to change the blinking speed or emission intensity of the LEDs 131-i and/or the fixation target 131c, or to select any of the LEDs 131-i and turn them on or cause them to blink (i.e., change the arrangement pattern of bright points).

It is possible to repeat the detection of the fixation state as described above and the controls based thereon at prescribed time intervals. As a result, it is possible to monitor the fixation state in real time and to incorporate the results thereof into controls in real time. It is thereby possible for the operator to know the fixation state in real time. Moreover, because the fixation state generally changes over time, it becomes possible to correct the fixation state in real time if it becomes poor.

### [Operations]

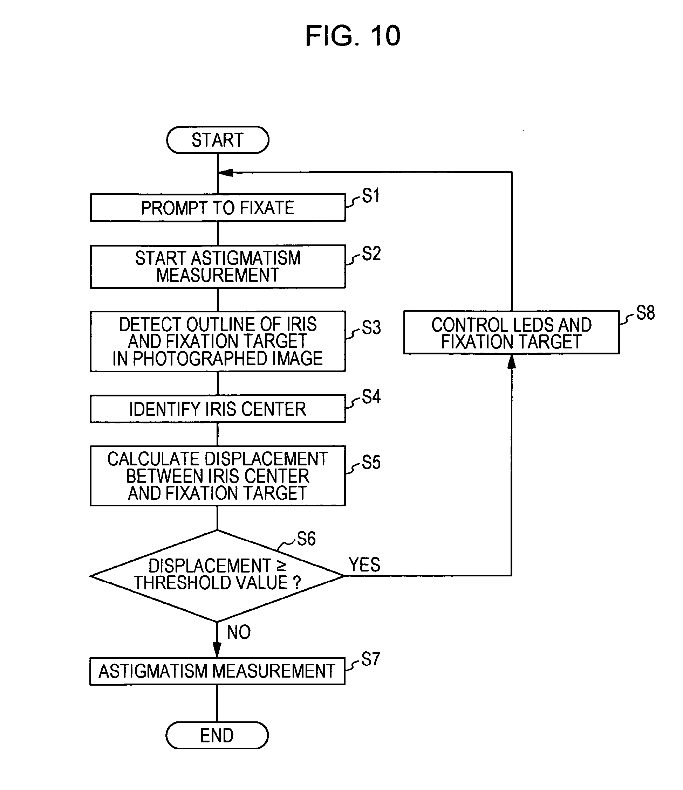

Operations of the microscope for ophthalmologic surgery 1 are described. Fig. 10 shows an example of operations of the microscope for ophthalmologic surgery 1.

First, the LEDs 131-i and the fixation target 131 c are turned on, and imaging of the patient's eye E using a TV camera 56 is started. Video signals from an imaging element 56a are sequentially input into the controller 60. The operator, etc. prompts the patient to direct their line of sight to the fixation target 131 c (S1). Then, the operator issues an instruction to start astigmatism measurement (S2). This instruction is issued via the operation part 82.

The controller 60 sequentially sends frames of the video signals from the imaging element 56a to the data processing part 90. Here, all of the frames may be sent, or selected frames may be sent. The displacement calculating part 92 analyzes each frame (photographed image) that is sent and detects the outline of the iris Eb of the patient's eye E as well as the fixation target 131c (S3).

Next, the displacement calculating part 92 obtains an approximate ellipsoid of the outline of the iris Eb detected in Step 3 and calculates the center of this approximate ellipsoid. The center of this approximate ellipsoid is considered to be the iris center Ec (S4).

Next, the displacement calculating part 92 calculates the displacement Δ of the iris center Ec obtained in Step 4 relative to the fixation target 131c obtained in Step 3 (S5). The displacement calculating part 92 sends data on the displacement Δ to the displacement determination part 61.

The displacement determination part 61 compares the absolute value of the displacement Δ (i.e., the amount of displacement) and a threshold value (S6). If the amount of displacement is less than the threshold value (S6: No), the controller 60 controls the astigmatism-information calculating part 91 to execute a process of calculating the astigmatism information (S7). As a result, astigmatism information of the patient's eye E is obtained. In this case, the processing ends here. In this case as well, the controls of the LEDs 131-i and the fixation target 131 c described previously may be performed.

If the amount of displacement is equal to or greater than the threshold value (S6: Yes), the presentation controller 62 controls the LEDs 131-i and the fixation target 131c (S8) and prompts the patient to fix his/her line of sight (S1), thereby causing the line of sight of the patient's eye E to move toward the fixation target 131c. Then, the above processes are repeated.

A configuration may be used in which, in Step 6, a notification is issued if the result is "No" a prescribed number of times. This notification may be issued by controlling the LEDs 131-i and/or the fixation target 131c, or may be issued via another means (message display, alarm sound, etc.).

### [Effects]

Effects of the microscope for ophthalmologic surgery 1 are described.

The microscope for ophthalmologic surgery 1 can perform astigmatism measurement of the patient's eye E using the projection-image forming part 13 installed on the microscope 6. The projection-image forming part 13 is provided with multiple bright points for astigmatism measurement (the LEDs 131-i) that are arranged in a ring, and the fixation target 131 c arranged inside this ring-shaped arrangement. Based on a photographed image of the patient's eye E in a state in which the LEDs 131-i and the fixation target 131c are projected thereto, the displacement calculating part 92 detects the fixation state of the patient's eye E relative to the fixation target 131 c. The controller 60 controls the LEDs 131-i and/or the fixation target 131 c based on the detection result of the fixation state.

Moreover, the controller 60 includes the displacement determination part 61 and the presentation controller 62. The displacement determination part 61 determines the quality of the fixation state based on the detection result of the fixation state. If the fixation state is determined to be poor, the presentation controller 62 controls the LEDs 131-i and/or the fixation target 131c so that the line of sight of the patient's eye E is oriented to the fixation target 131c.

Moreover, if the displacement determination part 61 determines that the fixation state is god, the controller 60 controls the astigmatism-information calculating part 91 and causes it to execute calculation of the astigmatism information of the patient's eye E.

As shown in Fig. 7A through Fig. 9C, etc., with the microscope for ophthalmologic surgery 1, even if the alignment is not correct, if the eye fixation is correct, astigmatism measurement is executed. This is a significant characteristic of the microscope for ophthalmologic surgery 1 that differs from conventional ophthalmologic devices. That is, with conventional microscopes for ophthalmologic surgery, because the operator moves the microscope as needed, it is therefore not possible in practice to perform alignment. This embodiment realizes suitable astigmatism measurement based on the assumption that alignment cannot be performed, and differs from conventional microscopes for ophthalmologic surgery and from other types of ophthalmologic devices in terms of perspective, configuration, operation and effect.

Moreover, the displacement calculating part 92 obtains the amount of displacement of the line of sight of the patient's eye E relative to the fixation target 131 c as the fixation state of the patient's eye E, and the controller 60 (the presentation controller 62) changes the details of the control of the LEDs 131-i and/or the fixation target 131 c in accordance with this amount of displacement.

Moreover, the displacement calculating part 92 obtains the direction of displacement of the line of sight of the patient's eye E relative to the fixation target 131 c as the fixation state of the patient's eye E, and the controller 60 (the presentation controller 62) changes the details of the control of the LEDs 131-i and/or the fixation target 131 c in accordance with this direction of displacement.

According to such microscope for ophthalmologic surgery 1, by controlling the LEDs 131-i and/or the fixation target 131c in accordance with the fixation state of the patient's eye E, it is possible to output information to the operator and/or the patient. In particular, if the fixation state is poor, it is possible to notify as such to the operator and to guide the line of sight of the patient. Consequently, it becomes possible to adjust the positional relationship between the patient's eye E and the optical system for astigmatism measurement in a suitable manner.

Moreover, according to the microscope for ophthalmologic surgery 1, if the fixation state is determined to be good, it is possible to swiftly shift to astigmatism measurement.

Moreover, according to the microscope for ophthalmologic surgery 1, because the details of the control of the LEDs 131-i and/or the fixation target 131 c can be changed in accordance with the amount of displacement or the direction of displacement (i.e., the amount or direction of fixation misalignment), it becomes possible to respond flexibly in accordance with the state of the fixation misalignment.

The configuration described above is nothing more than an example. Persons who intend to implement the present invention may make any changes, omissions, or additions within the scope of the present invention.

### [Explanation of Symbols]

1 Microscope for ophthalmologic surgery 5 Driving device
13 Projection image forming part
20 Illumination optical system
21 Illumination light source
30 Observation optical system
56a Imaging element
60 Controller
61 Displacement determination part
62 Presentation controller
81 Magnifying mechanism
82 Operation part
90 Data processing part
91 Astigmatism-information calculating part
92 Displacement calculating part
131-i LED
131c fixation target

## Claims

1. A microscope for ophthalmologic surgery comprising:
an optical system comprising an imaging optical system that photographs a patient's eye;
a main body part that stores at least part of the optical system;
an attachment that is attached to the main body part and has both multiple bright points for astigmatism measurement arranged in a ring and a fixation target arranged inside the ring-shaped arrangement;
a calculation part that calculates astigmatism information based on a first image obtained by using the imaging optical system to photograph the patient's eye while the multiple bright points are projected thereto;
a detection part that detects the fixation state of the patient's eye relative to the fixation target based on a second image obtained by using the imaging optical system to photograph the patient's eye while the multiple bright points and the fixation target are projected thereto; and
a controller that controls the multiple bright points and the fixation target based on the detection result of the fixation state.

2. The microscope for ophthalmologic surgery according to Claim 1,
wherein the controller
includes a determination part that determines the quality of the fixation state based on the detection result of the fixation state, and
if the fixation state is determined to be poor, controls the multiple bright points and the fixation target to orient the line of sight of the patient's eye to the fixation target.

3. The microscope for ophthalmologic surgery according to Claim 2, wherein if the determination part determines that the fixation state is good, the controller causes the calculation part to execute the calculation of the astigmatism information.

4. The microscope for ophthalmologic surgery according to any of Claims 1 through 3, wherein
the detection part detects the amount of displacement of the line of sight of the patient's eye relative to the fixation target as the fixation state, and
the controller changes the details of the control of the multiple bright points and the fixation target in accordance with the amount of displacement.

5. The microscope for ophthalmologic surgery according to any of Claims 1 through 4, wherein
the detection part detects the direction of displacement of the line of the of the patient's eye relative to the fixation target as the fixation state, and
the controller changes the details of the control of the multiple bright points and the fixation target in accordance with the direction of displacement.
